# EUROPEAN PATENT APPLICATION

(11) **EP 4 382 537 A1**
(43) Date of publication of application: **12.06.2024**
(21) Application number: 22849955.4
(22) Date of filing: 01.08.2022
(51) Int. Cl.: C07K 16/28, A61K 47/68, A61P 29/00, A61P 37/00

(54) **ANTI CD154 ANTIBODY AND USE THEREOF**

(30) Priority: 30.07.2021 KR 20210100482
(71) Applicant: Seoul National University R & DB Foundation, Seoul 08826 (KR); Ewha University-Industry Collaboration Foundation, Seoul 03760 (KR)
(72) Inventor: CHUNG, Jun Ho, Seoul 06521 (KR); CHOI, Kyung Ho, Seoul 02814 (KR); KIM, Sang Il, Redwood City, California 94603 (US); LEE, Young Jae, Namwon-si, Jeollabuk-do 55743 (KR); KIM, Su Jeong, Redwood City, California 94603 (US); PARK, Seo Ryeong, Seoul 02846 (KR); HWANG, Si Won, Seoul 04378 (KR); KANG, Dong Min, Seoul 03024 (KR); KIM, Su Ree, Seoul 04572 (KR)
(74) Representative: Stolmár & Partner Patentanwälte PartG mbB
(86) International application number: PCT/KR2022/011334
(87) International publication number: WO 2023/008982

(57) **Abstract**

The present invention relates to an antibody specifically binding to CD154, and particularly to an antibody including HCDR, which includes amino acid sequences of SEQ ID NOs: 1 to 3, and LCDR which includes amino acid sequences of SEQ ID NOs: 4 to 6; as well as a pharmaceutical composition for preventing or treating T cell-mediated autoimmune diseases or organ transplant rejection, which includes the antibody and a drug.

## Description

### [Technical Field]

The present invention relates to an antibody that specifically binds to CD154, and particularly to an antibody that recognizes CD154 expressed on the surface of activated T cells; as well as a pharmaceutical composition for preventing or treating T cell-mediated autoimmune diseases or organ transplant rejection, which includes the antibody and a drug.

### [Background Art]

Activation of T cells is an essential phenomenon in inflammatory diseases, autoimmune diseases, transplant rejection, etc., and requires costimulatory signals in addition to T cell receptor engagement. CD 154 of recently activated T cells binds to CD40 of antigen-presenting cells (APCs), thus to facilitate the expression of CD80 and CD86, and promote cytokine production. CTLA-4 Ig (abatacept, Orencia) and belatacept (Nulojix), which bind to CD80 and CD86 to inhibit the binding between CD80/CD86 of APCs and CD28 of T cells, effectively suppress T cell activation, thereby preventing the progression of autoimmune diseases (Nature Review Immunol, 2001, 1:220).

In addition to CD28, a number of T cell co-activation signals have been identified. However, the most important signaling pathway in disease treatment is a signal through an interaction between CD154 (CD40L) and CD40 in T cells. The CD154 expression level of CD4+ T cells is closely relevant to disease severity, clinical outcome, disease remission, and therapeutic effects of TNF inhibitors in patients with autoimmune arthritis (Cells, 2019, 8:927). Antibodies binding to CD154 may not only inhibit the interaction with CD40 but also eliminate activated T cells expressing CD154 through complement mediated cytotoxicity (CDC), thereby suppressing transplant rejection in an MHC-mismatched skin transplantation model (Nature Medicine, 2003, 3:1275). Since then, clinical development of antibodies that bind to CD154 has been actively carried out, such that BG9588, IDEC-131, ABI793, and the like have been developed. However, the development has been discontinued due to an occurrence of thromboembolism. The cause was found to be a problem that soluble CD154 secreted from activated platelets and the antibody form an immune complex, and then binds to FcyRIIa of platelets thus to activate the platelets (J Immunol, 2010, 185:1577). Thereafter, VIB4920, a CD154 blocker without Fc, is under clinical development for Sjogren's syndrome (NCT04129164). Further, as anti-CD40L antibodies, Darpirolizumab pegol (NCT04294667) and SAR441334 (INX-021; NCT04572841, NCT05039840, NCT04879628) are also under clinical development in various formats in order to suppress the induction of thromboembolism by inhibiting binding to Fc receptors.

Inhibitors of CD40, the ligand of CD154, are also being developed, but the clinical effects of the CD40 inhibitors are inferior to those of CD154 inhibitors. One of reasons for this result is because other ligands of CD154 such as CD11b also exist in addition to CD40 (Am J Transplant, 2020, 20:2216).

Therefore, it is necessary to develop an antibody capable of specifically targeting CD154 to induce specific death of CD154+ T cells while simultaneously inhibiting the interaction between CD154 and CD40.

### [Summary of Invention]

### [Problems to be Solved by Invention]

An object of the present invention is to provide an antibody capable of specifically targeting CD154 thus to specifically bind to CD154+ T cells.

In addition, another object of the present invention is to provide a pharmaceutical composition for preventing or treating T-cell mediated autoimmune diseases, which includes an anti-CD154 antibody.

Further, another object of the present invention is to provide a pharmaceutical composition for preventing or treating organ transplant rejection, which includes an anti-CD154 antibody.

### [Means for Solving Problems]

1. An antibody, including: a heavy chain which includes heavy chain complementary determine region 1 (HCDR1) having the amino acid sequence of SEQ ID NO: 1, HCDR2 having the amino acid sequence of SEQ ID NO: 2, and HCDR3 having the amino acid sequence of SEQ ID NO: 3; and a light chain which includes light chain complementary determine region 1 (LCDR1) having the amino acid sequence of SEQ ID NO: 4, LCDR2 having the amino acid sequence of SEQ ID NO: 5, and LCDR3 having the amino acid sequence of SEQ ID NO: 6.
2. The antibody according to the above 1, further including a single chain variable fragment (scFv) having the amino acid sequence of SEQ ID NO: 7 and the amino acid sequence of SEQ ID NO: 8.
3. The antibody according to the above 1, wherein the antibody binds to human CD154 (cluster of differentiation 154).
4. An antibody-drug conjugate in which a drug is conjugated to the antibody according to any one of the above 1 to 3.
5. The antibody-drug conjugate according to the above 4, wherein the drug is conjugated to the antibody through a linker or a secondary antibody.
6. The antibody-drug conjugate according to the above 4, further including a hapten.
7. The antibody-drug conjugate according to the above 6, wherein the hapten is continine.
8. The antibody-drug conjugate according to the above 4, wherein the drug is selected from the group consisting of monomethyl auristatin E (MMAE), mertansine (DM1), calicheamicins, pyrrolobenzodiazepine (PBD) dimer, alpha-amanitin (α-amanitin) and duocarmycin.
9. A pharmaceutical composition for preventing or treating T cell-mediated autoimmune diseases, including the antibody-drug conjugate according to any one of the above 4 to 8.
10. The composition according to the above 9, wherein the T cell-mediated autoimmune disease is selected from the group consisting of graft-versus host disease, rheumatoid arthritis, systemic lupus erythematous, Crohn's disease, multiple sclerosis, lupus nephritis, psoriasis (Ps), primary focal and segmental glomerular sclerosis, and immune thrombocytopenia.
11. A pharmaceutical composition for preventing or treating organ transplant rejection, including the antibody-drug conjugate according to any one of the above 4 to 8.

### [Advantageous effects]

The present invention may provide a novel antibody specifically binding to CD154. Preferably, it is usable as an antibody-drug conjugate in which a drug is conjugated to an anti-CD154 antibody, and the antibody-drug conjugate may be used as a pharmaceutical composition for preventing or treating T cell-mediated autoimmune diseases.

### [Brief Description of Drawings]

FIG. 1 illustrates confirmation of the binding of anti-CD154 antibody to CD154+ L cells by flow cytometry.
FIG. 2 illustrates confirmation of the binding of an anti-CD154 antibody containing LALA mutation to CD154+ L cells by flow cytometry.
FIG. 3 shows data of confirming a ratio of CD4+CD154+ T cells over time after CD4+ T cells were cultured in an active medium, and confirmation of the binding of CD4+CD154+ T cells and the anti-CD154 antibody by flow cytometry.
FIG. 4 illustrates results of confirming that the anti-CD154 antibody binds to CD154 thus to inhibit the function of CD154.
FIG. 5 illustrates confirmation of the blood half-life of the anti-CD154 antibody.
FIG. 6 illustrates confirmation of the intracellular internalization and localization of the anti-CD154 antibody.
FIG. 7 illustrates results of confirming that an anti-CD154 antibody-drug conjugate may kill CD154+ L cells.
FIG. 8 illustrates results of confirming that an anti-CD154 antibody-drug conjugate containing LALA mutation may kill CD154+ L cells.
FIG. 9 illustrates results of confirming that the anti-CD154 antibody-drug conjugate may kill CD154+ T cells.
FIG. 10 shows the amino acid sequence of the anti-CD154 antibody.
FIG. 11 shows the amino acid sequence of scFv region of a partially humanized antibody in which 13 chicken-derived residues were substituted with human-derived residues.
FIG. 12 shows the LCDR amino acid sequence and the HCDR amino acid sequence of the anti-CD154 antibody

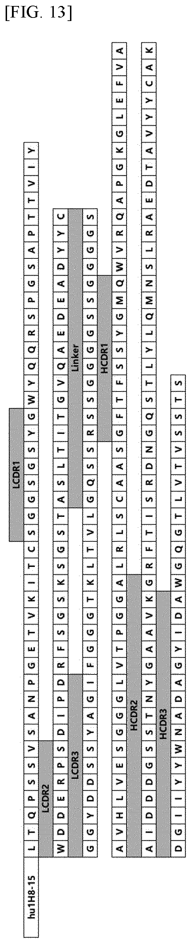

FIG. 13 shows the amino acid sequence of scFv region of a partially humanized antibody in which 25 chicken-derived residues were substituted with human-derived amino acid sequences.

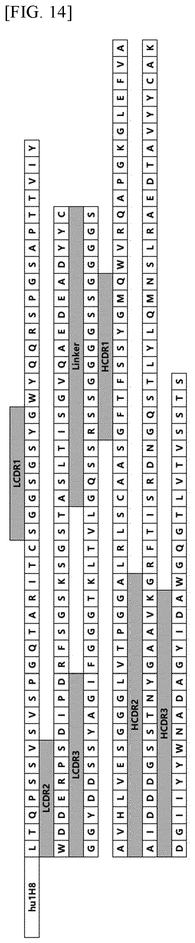

FIG. 14 shows the amino acid sequence of scFv region of a partially humanized antibody in which 31 chicken-derived residues were substituted with human-derived amino acid sequences.
FIG. 15 is a schematic diagram of an antibody-drug conjugate obtained by binding MMAE to an anti-CD154 antibody (IgG1) containing LALA mutation.
FIG. 16 shows data confirming whether there is a conjugation between the anti-CD154 antibody and an FcBP linker through HIC-HPLC.
FIG. 17 shows data confirming whether there is a conjugation between the anti-CD154 antibody and MMAE drug through HIC-HPLC.

### [Mode for Carrying out Invention]

Hereinafter, the present invention will be described in detail. Unless otherwise defined, all terms in this specification are the same as the general meanings of the terms understood by those skilled in the art to which the present invention pertains. Further, if the general meanings conflict with the meanings of the terms used in this specification, the terms follow the meanings used in the specification.

The present invention relates to an antibody, including: a heavy chain which includes heavy chain complementary determine region 1 (HCDR1) having the amino acid sequence of SEQ ID NO: 1, likewise, HCDR2 having the amino acid sequence of SEQ ID NO: 2 and HCDR3 having the amino acid sequence of SEQ ID NO: 3; and a light chain which includes light chain complementary determine region 1 (LCDR1) having the amino acid sequence of SEQ ID NO: 4, likewise, LCDR2 having the amino acid sequence of SEQ ID NO: 5 and LCDR3 having the amino acid sequence of SEQ ID NO: 6.

As long as the antibody of the present invention has HCDR amino acid sequences of SEQ ID NOs: 1 to 3, and LCDR amino acid sequences of SEQ ID NOs: 4 to 6, the remaining amino acid sequences are not limited. For example, it may be an antibody including a single chain variable fragment (scFv) in which the amino acid sequences of SEQ ID NO: 7 and SEQ ID NO: 8 are contained, but it is not limited thereto.

In the case of an antibody including the single chain variable fragment, the heavy chain variable region sequence and the light chain variable region sequence may be connected by a linker or the like. The linker sequence may be selected by those skilled in the art without limitation, and an example of the linker sequence may be the amino acid sequence of SEQ ID NO: 15, but it is not limited thereto.

The main regions of an antibody, which recognize a specific epitope of an antigen to form an antigen-antibody complex, such as the variable regions of the heavy and light chains, particularly complementarity determining regions (CDRs), contribute to the formation of this complex. Therefore, the present invention may include monoclonal antibodies, chimeric antibodies, humanized antibodies, etc. of the above antibody, which include the above-described variable regions of the inventive antibody, particularly, CDRs within the scope of the present invention. The humanized antibody may be, for example, an antibody including the amino acid sequence of SEQ ID NO: 9 and the amino acid sequence of SEQ ID NO: 10, or an antibody including the amino acid sequence of SEQ ID NO: 11 and the amino acid sequence of SEQ ID NO: 12. Alternatively, it may be an antibody including the amino acid sequence of SEQ ID NO: 13 and the amino acid sequence of SEQ ID NO: 14, but it is not limited thereto.

Further, so long as the variable regions of the monoclonal antibody of the present invention, particularly the CDRs are identical, an IgG subclasses of IgG₁ to IgG₄, etc. may also be included within the scope of the present invention. Further, the case where there is mutation in some of the amino acid sequences of the IgG subclass may also be included. For example, when LALA mutations (Leu234Ala/Leu235Ala) exist in the human IgG1 sequence, the binding of anti-Fc antibodies to the Fc region may be suppressed, thereby minimizing side effects such as thromboembolism. Further, the present invention may include functional fragments of antibody molecules as well as complete forms having two full-length light chains and two full-length heavy chains, so long as they have the binding characteristics described above. The functional fragment of the antibody molecule refers to a fragment having at least antigen-binding function, and may include Fab, F(ab'), F(ab')2 and Fv, etc., but it is not limited thereto.

Further, even if the antibodies of the present invention are bispecific, trispecific or multiple antibodies capable of binding to more antigens, any antibody may be included within the scope of the present invention regardless of the sequence, so long as at least one of the antigen-binding sites has the HCDR sequences of SEQ ID NOs: 1 to 3, and the LCDR sequences of SEQ ID NOs: 4 to 6. For example, it may be a bispecific antibody which has variable fragment (Fv) sequences specifically binding to CD154 and a hapten, respectively. In this case, an antibody-drug conjugate may be prepared by conjugating the hapten and a drug, etc., but it is not limited thereto.

The antibody of the present invention may bind to CD154 (cluster of differentiation 154). CD154 corresponds to a cell surface protein that is mainly expressed in activated CD4+ T cells, that is, CD4+ T cells capable of actively undergoing an immune response, and is not expressed in resting (in-activated) T cells. However, other than CD4+ T cells, the above antibody may also be expressed on the surface of activated CD 8+ T cells, natural killer (NK) cells, monocytes, basophils, eosinophils, or activated platelets. Therefore, the antibody of the present invention may correspond to an antibody that can distinguish the activated T cells and the resting T cells, and thus be selectively bound to surface proteins of the activated T cells. The antibody of the present invention may bind to CD154 of any one selected from the group including humans, rhesus monkeys *(Macaca mulatta,* Rhesus macaque), three-striped night monkeys *(Aotus trivirgatus),* Douroucouli, and soot mangabey *(Cercocebus atys*), but it is not limited thereto.

Further, the present invention relates to an antibody-drug conjugate in which a drug is conjugated to the antibody.

The antibody-drug conjugate of the present invention is capable of delivering a drug to a cell to which the antibody can be specifically bound. Specifically, the drug can be selectively delivered to cells expressing CD154 by conjugating the antibody of the present invention, which is capable of specifically binding to CD154, and the drug.

In the present invention, the conjugate between the antibody and the drug may be a direct bond or an indirect bond through a linker or a secondary antibody linked to the antibody, but it is not limited thereto. For example, it can be sufficient so long as the antibody and the drug can be jointly delivered to the target cells.

In the present invention, the secondary antibody refers to an antibody that specifically binds to the amino acid sequence of another antibody (a primary antibody) binding to an antigen, and corresponds to a terminology which is different from the primary antibody directly binding to a target antigen, in terms of the binding target. In the present invention, the linker is used to link a drug and an antibody. For example, a linker sequence is attached to Fc sequence of the antibody and then the linker sequence and the drug are linked to form an antibody-linker-drug. Alternatively, as mentioned above, the antibody may be constructed as a bispecific antibody and a hapten-linker-drug may be used so that it binds to a hapten-specific Fv region. Both the linker and the secondary antibody are used for conjugating the antibody of the present invention with the drug, and can be used according to any method used by those skilled in the art so long as it is intended to conjugate the antibody with the drug regardless of the types of the secondary antibody sequence or the linker.

In the present invention, the types of the drug may be selected by those skilled in the art according to the purpose without limitation. For example, in order to kill the activated T cells expressing CD154 on the surface, a drug may be selected from the group including monomethyl auristatin E (MMAE), mertansine (DM1), calicheamicins, pyrrolobenzodiazepine (PBD) dimer, alpha-amanitin (α-amanitin)and duocarmycin, and conjugated with the antibody. As mentioned above, the conjugation method may be selected from the known methods without limitation, and the types of the drug may vary depending on the purpose.

In the present invention, the antibody-drug conjugate may further include a hapten. In the present invention, the hapten is intended to be conjugated with a drug through the linker, and bind to Fv region having a binding site specific to the hapten in the antibody of the present invention so as to form a stable antibody-drug conjugate. Therefore, any hapten may be included within the scope of the present invention without limitation, so long as it corresponds to the above-described purpose. That is, an example of the hapten may include cotinine. However, it is not limited to thereto, and any molecule may be sufficient so long as it is non-immunogenic, but has antigenicity and is capable of forming hapten-specific antibodies.

When the antibody-drug conjugate of the present invention is used, due to the action mechanism in the antibody-drug conjugate (hereinafter referred to as ADC) manner, it is possible to prevent or treat T cell-mediated autoimmune diseases; or inhibit organ transplant rejection. The ADC is a method of injecting a drug by conjugating it to an antibody, and corresponds to a treatment method in such a principle that induces internalization of the antibody in a cell expressing an antigen, which specifically binds to the antibody, thereby delivering the drug into the cell and eventually cell-specifically delivering the drug. As mentioned above, after binding to T cells activated by the anti-CD154 antibody site, these are internalized into the cells and cause the death of T cells by the drug conjugated therewith, thereby demonstrating preventive or therapeutic effects on the above diseases.

Further, the present invention relates to a pharmaceutical composition for preventing or treating T cell-mediated autoimmune diseases, which includes the antibody-drug conjugate.

In the present invention, the T cell-mediated autoimmune disease refers to an autoimmune disease caused by induction of an immune response by T cells to a higher level than the normal state due to excessive proliferation or excessive activation of the T cells. For example, the T cell-mediated autoimmune disease may be any disease selected from the group including graft-versus host disease, rheumatoid arthritis, systemic lupus erythematous, Crohn's disease, multiple sclerosis, lupus nephritis, psoriasis (Ps), primary focal and segmental glomerular sclerosis, and immune thrombocytopenia. However, it is not limited thereto.

Further, the present invention relates to a pharmaceutical composition for preventing or treating organ transplant rejection (transplantation rejection), which includes the antibody-drug conjugate.

The antibody-drug conjugate of the present invention may inhibit organ transplant rejection by suppressing the immune response of the activated T cells involved in an organ transplantation process or rejection after organ transplantation.

The pharmaceutical composition of the present invention is formulated into a unit dosage form suitable for administration into the body of a patient according to any conventional method in the pharmaceutical field, preferably in the form of a formulation useful for the administration of protein drugs, and may be administered by a parenteral administration route including intravenous, intramuscular, intraarterial, intramedullary, intrathecal, intraventricular, pulmonary, transdermal, subcutaneous, intraperitoneal and nasal according to any administration method commonly used in the art, but it is not limited thereto.

Formulations suitable for the purpose described above are preferably preparations for parenteral administration, for example, injections such as ampoules for injection, infusions, and sprays such as hypospray. In the case of a formulation for injection or infusion, it may take the form of a suspension, solution or emulsion, and may also include formulation agents such as a suspending agent, a preservative, a stabilizer and/or a dispersing agent. Alternatively, the antibody molecule may be formulated in a dry form that can be reconstituted with an appropriate sterile liquid prior to use.

As an active ingredient of the pharmaceutical composition of the present invention, the antibody may be administered at 0.01 to 50 mg/kg body weight per day, and preferably 0.1 to 20 mg/kg body weight, once or divided into several times to mammals including humans. However, it is to be understood that the actual dosage of the active ingredient should be determined depending on various factors such as the disease to be prevented or treated, the severity of the disease, the route of administration, the patient's weight, age and sex, drug combination, reaction sensitivity, and tolerance/response to treatment or the like. Accordingly, the above dosages do not limit the scope of the present invention in any way.

Hereinafter, the present invention will be described in more detail by way of the following examples in order to concretely describe the present invention.

### Example 1. Establishment of anti-CD154 antibody (anti-hCD154)

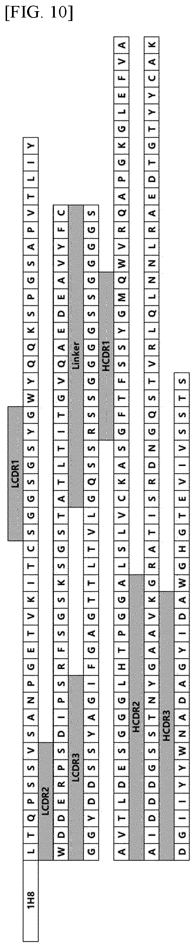

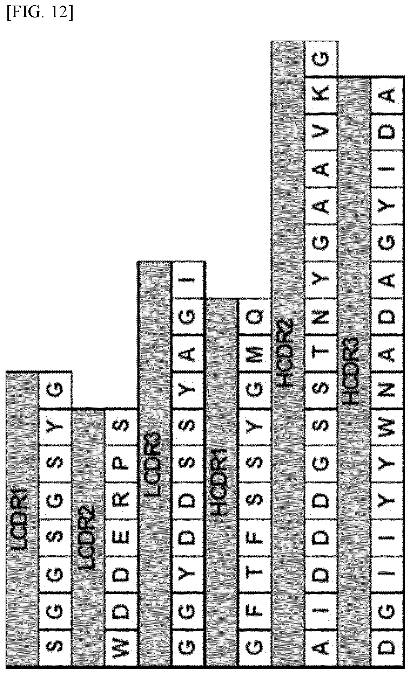

To prepare a monoclonal antibody against human CD154, an antibody binding to human CD154 expressed on the cell membrane surface was developed from an antibody library that was prepared after vaccination with human CD154 in chickens. The specific protocol was performed according to a previously established method. CDR sequences of heavy and light chains of the antibody are shown in FIG. 12, and variable region sequences are shown in FIG. 10.

Then, in order to prepare a fusion antibody in which the anti-CD154 region and the anti-cotinine scFv (single chain variable fragment) are bound, a vector was prepared wherein the terminal regions of the light and heavy chains of the antibody are bound with the anti-cotinine ScFv by a linker (Gly-Gly-Gly-Gly-Ser)₄, followed by transfecting the same into HEK293F cells (Invitrogen) using 25-kDA linear polyethyleneimine (Polyscience, Warrington, PA, USA) according to a conventionally known method. Then, using protein A agarose beads (RepliGen, Waltham, MA, USA), the anti-hCD154 x cotinine scFv-hCκ-scFv antibody was isolated through chromatography.

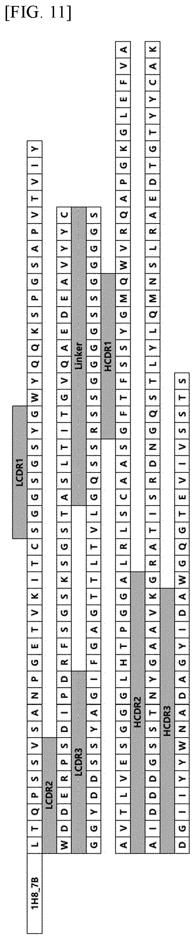

Further, in order to prepare IgG containing LALA mutation during antibody humanization, Leu234Ala and Leu235Ala mutations were induced in human IgG1. Thereafter, a vector was prepared through genetic recombination wherein the heavy and light chains of the anti-hCD154 antibody were linked to IgG1 heavy chain constant region containing LALA mutation and Ig kappa light chain constant region, respectively. Then, the antibody was isolated in the same manner as described above, thereby producing anti-hCD154 (1H8-7B) IgG1 (LALA) antibody (FIG. 11).

### Example 2. Confirmation of hCD154 binding ability of anti-CD154 antibody (in vitro)

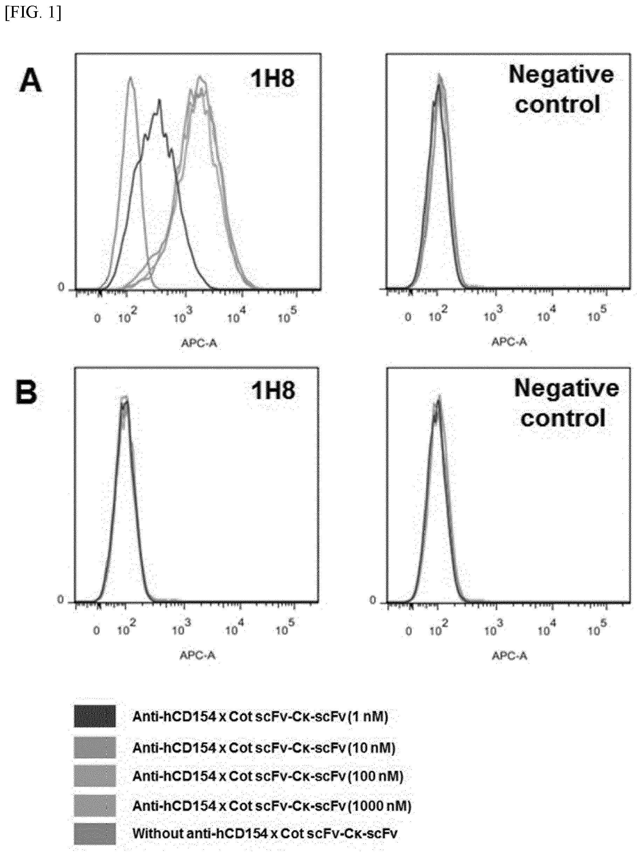

In order to determine the binding ability of the antibody with hCD154, the anti-hCD154 x cotinine scFv-hCκ-scFv fusion protein prepared in Example 1 above was treated with hCD154-expressing L cells or non-expressing L cells, followed by flow cytometry. Specifically, L cells (hCD154-) or L cells (hCD154+) were treated with serially diluted anti-hCD154 x cotinine scFv-hCκ-scFv fusion protein, and incubated, followed by measuring an amount of anti-hCD154 x cotinine scFv-hCκ-scFv fusion protein bound to cells using APC-conjugated anti-Cx antibody. Each sample was analyzed using 10,000 cells. As a result, it was confirmed that CD154+ L cells bind to 1H8 antibody-activated human T cells (FIG. 1).

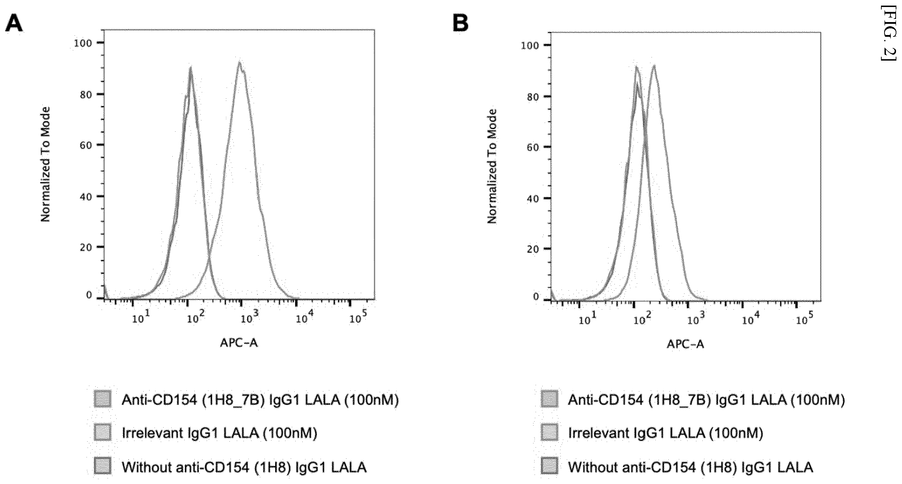

Further, an experiment was also implemented for anti-hCD154 (1H8_7B) IgG1 (LALA) containing LALA mutation in the same method, and it was confirmed that it could bind to human CD154 expressed on the surface of L cells as in the previous antibody (FIG. 2).

### Example 3. Confirmation of CD154 expression in human activated CD4+ T cells and confirmation of binding ability of anti-CD154 antibody

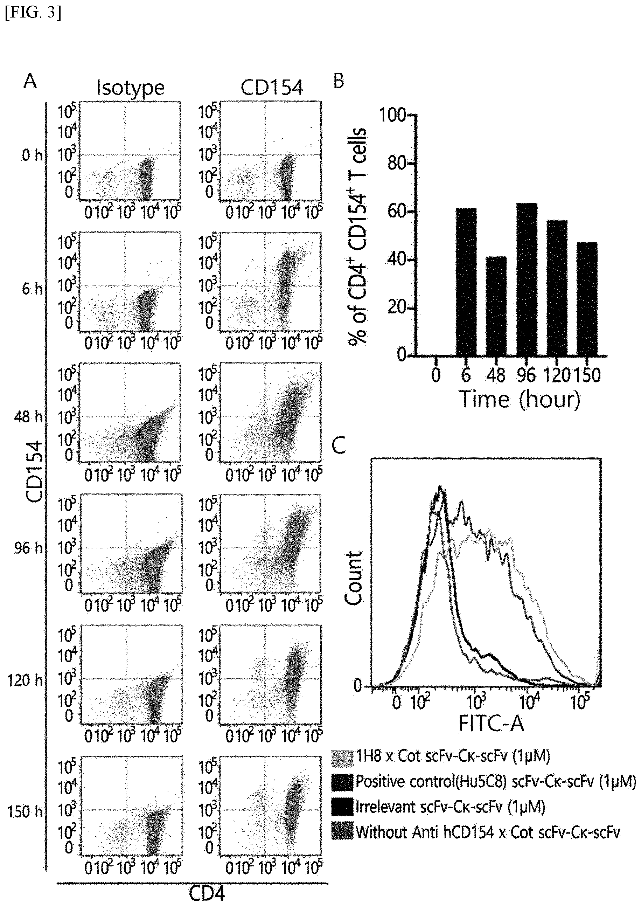

Primary human CD4+ T cells were activated by treatment with anti-CD28 and anti-CD3 antibodies for 3 days and cultured under human IL-2 for 2 days. Immediately after activation of the corresponding cell line, hCD154 expression levels were measured using a PE-labeled mouse anti-hCD154 antibody and a FITC-labeled mouse anti-CD4 antibody at 6 h, 48 h, 96 h, 120 h, and 150 h, respectively. As a result, it was confirmed that about 60% of the entire cell line were CD4+/CD 154+ T cells after 6 h. Further, it was confirmed that about 50% of the double-positive cells were present on average even up to 150 hours thereafter (FIGS. 3A and 3B). The analysis was performed at each time point using 10,000 cells.

According to the above results, primary human CD4+ T cells 6 hours after T cell activation were treated with the anti-hCD154 x cotinine scFv-hCκ-scFv antibody of Example 1, and an amount of anti-hCD154 x cotinine scFv-hCκ-scFv fusion protein bound to the cells was measured using APC-conjugated anti-Cx antibody, thus to determine whether the antibody was bound to T cells. As a result, it was confirmed that the 1H8 antibody binds to the activated CD4+ T cells (FIG. 3C).

### Example 4. Confirmation of CD154-CD40 binding inhibitory ability of anti-CD154 antibody

In the case where human CD154 or CD154-expressing L cells and CD40+ Daudi cells are co-cultured, by paying attention to the fact that Daudi cells express CD80 and CD86, anti-hCD154 x cotinine scFv-hCκ-scFv fusion protein (2,500 nM) was mixed with the recombinant HA-tagged hCD 154 protein (250 nM), and used for treatment of Daudi cells. Further, Daudi cells were treated along with CD154+ L cells or anti-hCD154 x cotinine scFv-hCκ-scFv fusion protein (30 nM). Then, the expression levels of CD80 and CD86 were measured with PE-labeled mouse anti-CD80 antibody and FITC-labeled mouse anti-CD86 antibody.

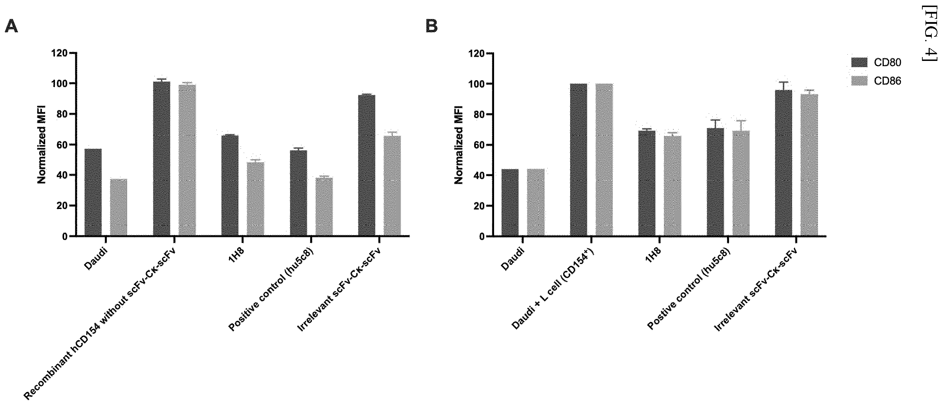

As a result, it was confirmed that the expression levels of CD86 and CD80 were increased when recombinant HA-tagged hCD154 protein was treated or when Daudi cells were treated with CD154+ L cells, whereas the margin of increase in the expression levels was considerably reduced when anti-hCD154 x cotinine scFv-hCκ-scFv antibody were used together for treatment. Therefore, it was demonstrated that the anti-CD154 antibody could effectively inhibit the binding of CD154 and CD40 (FIG. 4).

### Example 4. Confirmation of half-life of anti-CD154 antibody in blood

NSG mice (n=4) were intravenously injected with 500 µg of anti-hCD154 x cotinine scFv-hCκ-scFv. Thereafter, blood samples were collected from the orbital vein at various time intervals, and the concentration of anti-hCD154 x cotinine scFv-hCκ-scFv in blood was measured through ELISA. Specifically, a microtitler plate coated with recombinant hCD154 protein was incubated with blood or a standard solution, and measurement was conducted using an HRP-conjugated anti-human CK antibody as a probe and TMB as a substrate. All experiments were repeated 3 times, and data are presented as mean and standard deviation.

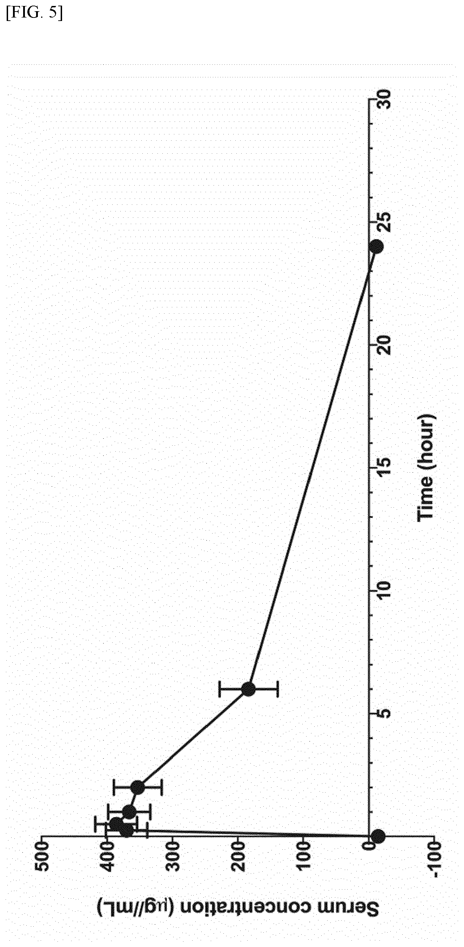

As a result, it was confirmed that the antibody could be maintained without decomposition until about 20 hours after injection, and the half-life was around 6 hours (FIG. 5).

### Example 5. Preparation of anti-CD154-drug conjugate (anti-hCD154(1H8-7B) IgG1 (LALA)-MMAE)

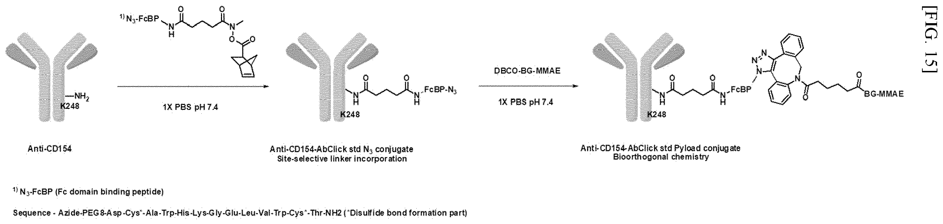

Anti-hCD154 (1H8-7B) IgG1 (LALA) mentioned in Example 1 was bound with MMAE to prepare an antibody-drug conjugate (FIG. 15).

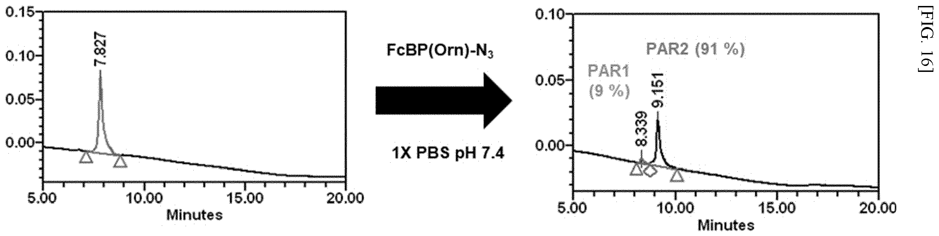

Specifically, an FcBP(Orn)-N₃ linker was synthesized, and for azide introduction at K248 position of the antibody on pH 7.4 1 x phosphate buffered saline (PBS) buffer, 3.0 equivalents (30.6 nmol) per antibody (1.53 mg, 10.2 nmol) of the compound FcBP(Orn)-N₃ were put into the reaction solution, followed by performing the reaction. The reaction was performed at room temperature for 6 hours, and the entire process including termination of the reaction was monitored through HIC-HPLC. As a result, after the reaction, it was confirmed that there was a reaction between the anti-CD154 antibody and the FcBP linker as shown in FIG. 16. Purification was conducted by dialysis (Dialysis, pH 7.4 1 x PBS) three times to secure CD154 azide (yield = 91%).

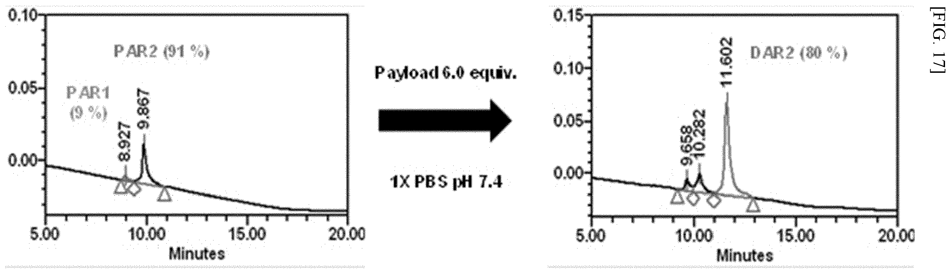

Then, an antibody-drug complex was prepared using CD154-N₃ into which two molecules of azide were introduced by the compound FcBP(Orn)-N₃. The reaction was carried out using an amount of 1.7 mL (10.2 nmol) at a concentration of 0.9 mg/mL, and conjugation of DBCO-BG-MMAE drug was attempted for biorthogonal chemistry with azide conjugated to the antibody. 6.0 equivalents (61.2 nmol) of the drug based on the antibody were used, and the conjugation reaction was conducted in pH 7.4 1 x PBS buffer at room temperature for 3 hours. The conjugation reaction was observed and confirmed through HIC-HPLC (FIG. 17). Purification was conducted by dialysis (Dialysis, pH 7.4 1 x PBS) three times to secure Anti-CD154-MMAE ADC.

### Example 6. Confirmation of intercellular flow of anti-CD154-drug conjugate

hCD154+ L cells and hCD154- L cells were incubated along with the anti-hCD154 x cotinine scFv-hCκ-scFv fusion protein. Antibodies bound to the cell surface were removed, the cells were fixed and stained with FITC-conjugated anti-human CK antibody (green). To detect early endosomes, the cells were treated along with rabbit anti-Rab5 antibody and incubated, followed by treatment using Alexa Fluor 546-conjugated goat anti-rabbit IgG (red). Cell nuclei were stained using DAPI.

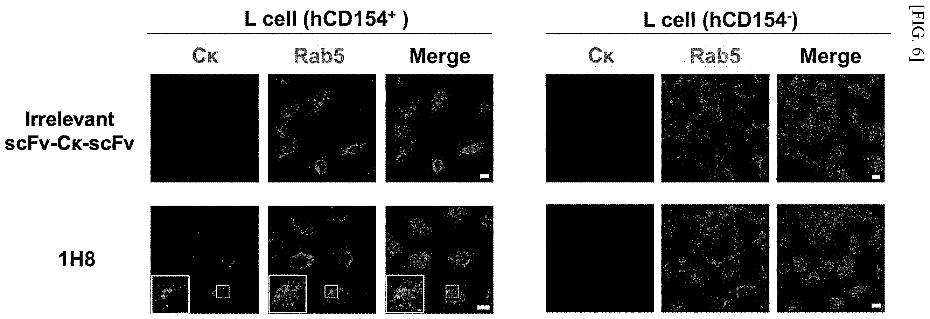

As a result, it was confirmed that the anti-hCD154 x cotinine scFv-h Cκ-scFv fusion protein and the early endosome were co-located in the cell (FIG. 6).

### Example 7. Confirmation of cell killing ability of anti-CD154-drug conjugates

An antibody-drug conjugate (anti-hCD154 x cotinine scFv-hCκ-scFv; continine-duocarmycin) prepared by treating anti-hCD154 x cotinine scFv-hCκ-scFv with cotinine-duocarmycin, or the antibody-drug conjugate of Example 5 (anti-hCD154(1H8-7B) IgG1 (LALA)-MMAE) was treated on hCD154+ L cells or hCD154- L cells, respectively. Thereafter, a cytotoxicity assay was performed to measure the degree of cell death depending on the treatment concentration.

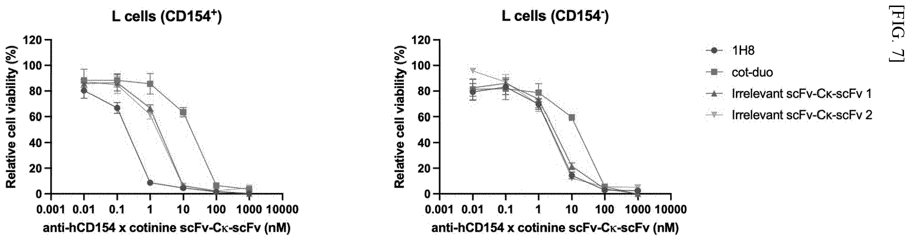

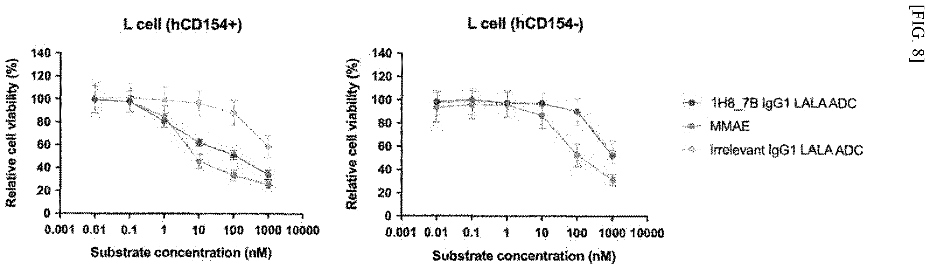

As a result, it was confirmed that both the antibody-drug conjugate in which anti-hCD154 x cotinine scFv-hCκ-scFv was bound to cotinine-duocarmycin, and the antibody-drug conjugate in which MMAE was bound to anti-hCD154 (1H8-7B) IgG1 (LALA) could kill CD154+ L cells in which human CD154 was artificially expressed (FIGS. 7 and 8).

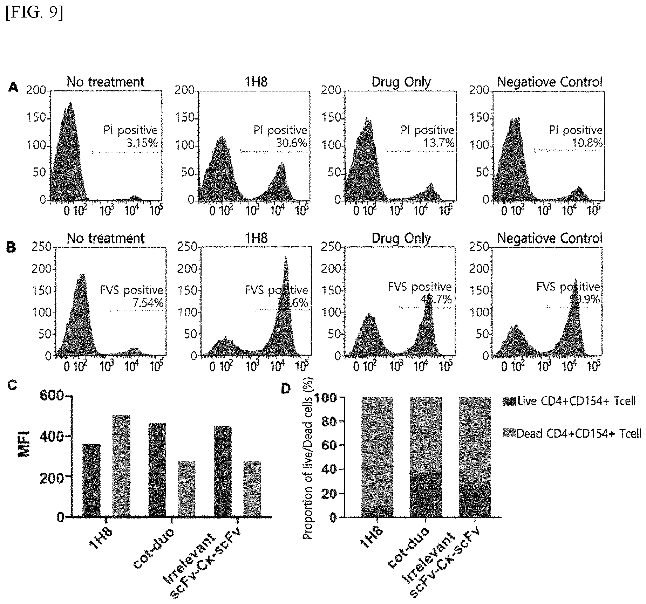

Further, through flow cytometry, the antibody-drug conjugate including anti-hCD154 x cotinine scFv-hCκ-scFv and cotinine-duocarmycin bound together was confirmed to have the ability to kill CD4+ T cells. The antibody-drug conjugate was treated on CD4+ T cells for 48 hours, and a ratio of surviving cells/dead cells was measured using propidium iodide. As a result of the measurement, it was confirmed that, compared to only drug treatment (13.7%), the death rate of T cells was increased when treated with the antibody-drug conjugate (30.6%) (FIG. 9A). In addition, even when measured through fixable viability stain (FVS) staining, it was confirmed that the experimental group (74.9%) containing CD4+ T cells treated with the antibody-drug conjugate for 60 hours showed higher killing ability (74.9%) than the case of treating only with the drug (48.7%) (FIG. 9B). Further, when the mean fluorescence intensity (MFI) and cell survival/death ratio of FVS in the sample (treated for 60 hours) were separately indicated, there is no significant difference in the dead cell rates when treated only with scFv-hCk-scFv to which duocarmycin or hCD154 is not bound alone. On the other hand, when treated with the antibody-drug conjugate, it was confirmed that the dead cell rate was significantly increased, thereby demonstrating that the above effect is due to the antibody-drug conjugate itself. Further, it was also confirmed that the antibody-drug conjugate could selectively kill cells expressing hCD154.

## Claims

1. An antibody, comprising:
a heavy chain which comprises heavy chain complementary determine region 1 (HCDR1) having the amino acid sequence of SEQ ID NO: 1, HCDR2 having the amino acid sequence of SEQ ID NO: 2, and HCDR3 having the amino acid sequence of SEQ ID NO: 3; and
a light chain which comprises light chain complementary determine region 1 (LCDR1) having the amino acid sequence of SEQ ID NO: 4, LCDR2 having the amino acid sequence of SEQ ID NO: 5, and LCDR3 having the amino acid sequence of SEQ ID NO: 6.

2. The antibody according to claim 1, further comprising a single chain variable fragment (scFv) having the amino acid sequence of SEQ ID NO: 7 and the amino acid sequence of SEQ ID NO: 8.

3. The antibody according to claim 1, wherein the antibody binds to human CD 154 (cluster of differentiation 154).

4. An antibody-drug conjugate in which a drug is conjugated to the antibody according to any one of claims 1 to 3.

5. The antibody-drug conjugate according to claim 4, wherein the drug is conjugated to the antibody through a linker or a secondary antibody.

6. The antibody-drug conjugate according to claim 4, further comprising a hapten.

7. The antibody-drug conjugate according to claim 6, wherein the hapten is continine.

8. The antibody-drug conjugate according to claim 4, wherein the drug is selected from the group consisting of monomethyl auristatin E (MMAE), mertansine (DM1), calicheamicins, pyrrolobenzodiazepine (PBD) dimer, alpha-amanitin (α-amanitin) and duocarmycin.

9. A pharmaceutical composition for preventing or treating T cell-mediated autoimmune diseases, comprising the antibody-drug conjugate according to any one of claims 4 to 8.

10. The composition according to claim 9, wherein the T cell-mediated autoimmune disease is selected from the group consisting of graft-versus host disease, rheumatoid arthritis, systemic lupus erythematous, Crohn's disease, multiple sclerosis, lupus nephritis, psoriasis (pSS), primary focal and segmental glomerular sclerosis, and immune thrombocytopenia.

11. A pharmaceutical composition for preventing or treating organ transplant rejection, comprising the antibody-drug conjugate according to any one of claims 4 to 8.
